# EUROPEAN PATENT APPLICATION

(11) **EP 4 353 217 A1**
(43) Date of publication of application: **17.04.2024**
(21) Application number: 22806890.4
(22) Date of filing: 06.05.2022
(51) Int. Cl.: A61H 35/04, A61M 15/08, A61M 11/06, A61M 3/02, A61M 5/31, B05B 11/02

(54) **DOSING NOZZLE**

(30) Priority: 14.05.2021 ES 202130989 U
(71) Applicant: Delpuy Baquero, Gerard, 08320 El Masnou (Barcelona) (ES); Fadurdo Orellana, Roger, 08320 El Masnou (Barcelona) (ES)
(72) Inventor: Delpuy Baquero, Gerard, 08320 El Masnou (Barcelona) (ES); Fadurdo Orellana, Roger, 08320 El Masnou (Barcelona) (ES)
(74) Representative: Isern Patentes y Marcas S.L.
(86) International application number: PCT/ES2022/070280
(87) International publication number: WO 2022/238604

(57) **Abstract**

The present invention relates to a dosing nozzle, and more specifically to a fluid dosing nozzle for applying a fluid inside a user's nasal cavities, said nozzle comprising a flange with grooves to relieve potential excess pressure and thus avoid damaging the inside of a user's nasal cavities.

## Description

### Technical field of the invention

The present invention is directed to a dispensing nozzle, and more specifically to a fluid dispensing nozzle for applying a fluid inside user's nasal cavities, comprising a rim with slits to relieve possible excess pressure and thus avoid damaging the inside of a user's nasal cavities.

### Backgrounds of the invention

Mucus is a defense mechanism of the body against infections, very common in babies and children up to 6 years old, but also present in young people and adults. Generally, when a pathogen or a foreign body comes into contact with the respiratory tract, the body generates mucus that traps the infectious agents so that they can be expelled to the outside either by coughing or sneezing. During fall and winter, cold temperatures help develop respiratory infections. The mucus itself is not harmful, but it is very annoying and uncomfortable, especially for children, interrupting sleep or eating, and in excess it could clog the nasal cavities.

Even if there is no need for drug treatment, there are methods to mitigate excess mucus. The most effective and safe method, although uncomfortable, is to rinse the nose with a physiological saline solution, dissolving the mucus and helping to extract it. In addition to excess mucus, a person may want to clean the inside of the nose for other reasons, such as allergies, cleaning after administering medication with a nebulizer, congestion, etc.

It is currently possible to treat mucus with existing commercial products; Single-dose containers or large bags of saline solution are generally readily available from which the saline solution can be drawn with a syringe and subsequently applied to the user's nasal passages. Single-dose saline solution containers are very expensive and not very sustainable from an environmental point of view, which is why the use of syringes is preferred to relieve excess mucus because it is more sustainable and econom ic.

Unfortunately, the nozzles of conventional syringes do not fit properly to the shape of the nose, and the solution can spill without clearing the nostril, or produce harmful excess pressure inside the nasal cavities. Additionally, the nozzles, or outlet portions, of conventional syringes are pointed, and could injure the inside of the user's nose if the syringe is handled carelessly or if the user moves suddenly.

Devices have been developed to try to solve these drawbacks, but none of them is able to solve these problems satisfactorily, so there is still wide margin for improvement in the current technology field.

### Description of the invention

The present invention proposes a solution to the above problems by means of a dosing nozzle as defined in the independent claim, in particular by means of an ergonomic nozzle adaptable to the outlet portion of a syringe to apply a pressurized fluid in a controlled manner by the action of the syringe, preferably a saline solution, into the nostril of a user in order to provide a means for rinsing the mucus generated in the nasal cavity, without damaging the user's nose by overpressure or by impact of the end of a syringe.

In an inventive aspect, the invention provides a dispensing nozzle configured for applying into a nasal cavity of a pressurized fluid from a syringe, wherein the nozzle has a substantially tubular configuration, and comprises:
a first end with at least a first opening,
a second end with at least a second opening, and
a side wall extending between the first end and the second end, where the first opening and the second opening they are in fluid communication with each other;
the nozzle being characterized in that:
   the first end comprises an elastically deformable rim arranged around the first opening, and configured to establish a tight seal between the first end and the outside of the nose,
   the rim comprises at least one slit extending between the first opening and an outer edge of the rim, the slit being configured to relieve an excess pressure inside of a user's nasal cavities,
   the second end is elastically deformable and is configured to connect the second opening tightly with an outlet portion of a syringe, and the side wall comprises at least one flange configured to push or pull the nozzle in order to connect or disconnect the second opening to the outlet portion of the syringe.

Throughout this document it will be understood that the nozzle is a device adapted to conduct a fluid between the exit portion of a syringe and the interior of the nostril of a user, and consequently a fluid path is established from the second opening to the first opening. Syringe should preferably be understood as a conventional syringe with a body, an outlet portion and a plunger. By elastically deformable material means a flexible material or one that behaves in an elastic regime under normal operating conditions. By rim should be understood a substantially flat member surrounding the first opening and preferably arranged perpendicular to the side wall. By slit should be understood a recess or groove in a surface of the rim, preferably a surface further away from the second end. By outer edge of the rim, a peripheral portion of the rim should be understood.

In other embodiments, instead of an outlet portion of a syringe, an outlet portion of a single-dose container of a fluid or a connector member for infusion for healthcare use, such as a connector of a drip infusion device or a neti pot is connected to the nozzle.

Advantageously, the configuration of the nozzle allows the controlled dosage or application of a fluid in the nostril, or nasal cavity, of the user to dissolve and eliminate excess mucus without damaging said cavity with ease by means of a simple nozzle. In addition, the nozzle allows a good seal between the nozzle and the nose, while being safe from overpressure and can be used with any size of conventional syringe and any fluid.

Also advantageously, the nozzle solves at least the following problems of conventional devices:
- Lack of comfort: rinsing the solution directly with the mouth of the syringe is uncomfortable and may cause the solution to spill. An ergonomic nozzle will adapt better to the geometry of the nose and prevent leaks with a good seal. Different designs will suit different nose shapes.
- Efficiency: leaks reduce the effectiveness of the wash, preventing the fluid from reaching the bottom of the nose. The nozzle will securely seal the nose, whereby all amount of the solution introduced can reach all regions of the nose.
- Safety: good sealing will require less pressure on the fluid to reach the entire interior of the nasal cavity. In addition to preventing risk of injury and being less intrusive to the subject's nose, the nozzle contacts the outer edge of the nose and prevents the tip of the syringe or other sharp flange from hitting the septum or wall of the nasal cavity. This, combined with some slits, will avoid the risk of injury.
- Ease of cleaning: the nozzle is easy to disconnect from the syringe and allows cleaning of the nozzle, thanks to the flanges on the side wall of the nozzle.
- Flexibility: the nozzle can adapt to different sizes of conventional syringes, such as 5 ml or 10 ml, depending on the preferred use.

Also advantageously, the nozzle can be used to apply a medicine to the nasal cavity of a user.

In a particular embodiment, the rim can be detached from the rest of the nozzle and interchangeable with rims of different sizes and shapes. Advantageously, the adaptation of the rim to different sizes of the nose allows the nozzle to be used by users of any age and physiognomy.

In a particular embodiment, the nozzle is formed by an integral piece. Advantageously, a one single-piece nozzle can be manufactured economically and simply by conventional means, such as by injection or molding.

In a particular embodiment, the nozzle has substantially axial symmetry with respect to a longitudinal axis passing through the first opening and the second opening. Advantageously, the axial symmetry of the nozzle facilitates flow through the fluid path.

In a particular embodiment, the nozzle comprises two substantially elongated flanges arranged perpendicularly or obliquely with respect to the longitudinal axis of the nozzle. Advantageously, the flanges allow the nozzle to be pushed or pulled with the fingers to connect, disconnect or otherwise manipulate it.

In a particular embodiment, the nozzle is made of an elastically deformable material. Advantageously, the elastically deformable material allows on the one hand to establish a tight connection with exit portions of syringes of various sizes, and on the other hand to establish a tight contact between the nozzle and the nose of a user. In a particular embodiment, the nozzle is made up of rubber, food grade silicone or natural rubber.

In a particular embodiment, the rim comprises a recess in a portion of its outer edge. By "recess" means a chamfered portion of the rim, resulting from sectioning a part of the rim; The recess preferably has a complementary configuration to the part of the face called the subnasal, nasolabial, or philtrum groove, and advantageously allows better coupling of the nozzle to the user's nasal cavities or nostril.

In a particular embodiment, the side wall comprises a narrowing. By "narrowing" means a portion of the nozzle between the first and second ends with a diameter, or other corresponding dimension, less than the diameter, or corresponding dimension, of the rest of the nozzle. Advantageously, the narrowing allows the nozzle to be adapted to the anatomy of babies, whose positioning area at the mouth of the nostril is much smaller than in child and/or adult users.

These and other characteristics and advantages of the invention will be evident from the following description of the preferred, but not exclusive, embodiments, which are illustrated by way of non-limiting example in the accompanying drawings.

### Brief description of the drawings

Figures 1a, 1b.- These figures show a perspective and section view of a preferred embodiment of the nozzle, respectively.
Figures 2a, 2b.- These figures show a perspective and section view of the nozzle connected to a syringe, respectively.
Figure 3.- This figure shows perspective and section views of another preferred embodiment of the nozzle, also connected to a syringe.
Figure 4.- This figure shows a perspective and sectional view of another preferred embodiment of the nozzle, also connected to a syringe.

### Detailed description of an exemplary embodiment

In the following detailed description, numerous specific details are set forth in the form of examples to provide a thorough understanding of the relevant teachings. However, it will be apparent to those skilled in the art that the present teachings can be implemented without such details.

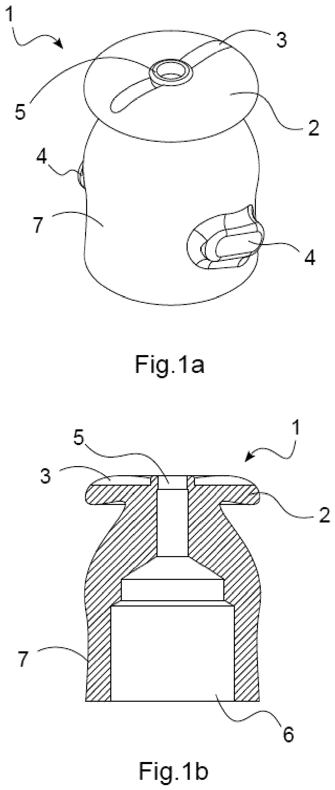

Figure 1a shows a perspective view of a preferred embodiment of the nozzle (1). In this example, the nozzle (1) has substantially axial symmetry and is substantially bell-shaped, where the first end, corresponding to the shoulder of the bell, is shown in the upper part of the figure, and the second end, shown in the lower part of the figure, corresponding to the foot of the bell. Surrounding the first opening (5) is a rim (2) with a disk configuration that has two slits (3) that extend radially from the first opening (5) to a perimeter portion of the rim (2) to relieve excess pressure. pressure during the application of the fluid with the nozzle (1). The side wall (7) comprises two elongated flanges (4) arranged obliquely with respect to the longitudinal axis of the nozzle (1); These flanges (4) allow the fingers to be pressed or supported on the nozzle (1), for example, to manipulate it or connect it to a syringe (10), preferably a 5 or 10 ml conventional syringe (10). In other exemplary embodiments, not shown in the figures, it is connected to the nozzle (1) a connector of a drip infusion set, a nasal Iota, or an outlet portion of a single-dose saline container.

Figure 1b shows a section view of the nozzle (1) according to the preferred embodiment shown in Fig. 1a, in which you can see the side wall (7) of the nozzle (1), the first opening (5), the second opening (6) and the rim (2). In this view it can be seen that the fluid conduit between the second (6) and the first opening (5) has a configuration corresponding to the outlet portion or mouth of a syringe (10), and in particular comprises a substantially cylindrical portion, configured to fit around an end of the syringe tube (10), a substantially conical portion, configured to fit around the cone of the syringe (10), and a substantially cylindrical portion, configured to fit around the pivot of the syringe (10).

In the embodiment shown in the figures, the nozzle (1) is formed by a single integral piece of natural rubber, manufactured by conventional means, and the second end is configured to fit mouths of syringes (10) of different diameters thanks to the behavior elastic of the material with which the nozzle (1) is made. In other embodiments, the nozzle (1) is made of a substantially rigid material. On the other hand, the rim (2) of the first end adjusts to the shape of a user's nose, and allows establishing a watertight connection between the first opening (5) and the nasal cavity, preventing the fluid from spilling out of the nasal cavity; Furthermore, the rim (2) prevents the first end from impacting the inside of the nostril and causing injuries inside it. Finally, in the event of excess fluid pressure produced by the syringe (10), the slits (3) allow part of the fluid from inside the nasal cavity to be evacuated to relieve excess pressure and prevent injuries. In other embodiments, not shown in the figures, the rim (2) is a separable piece that can be interchanged with a rim (2) of a different size.

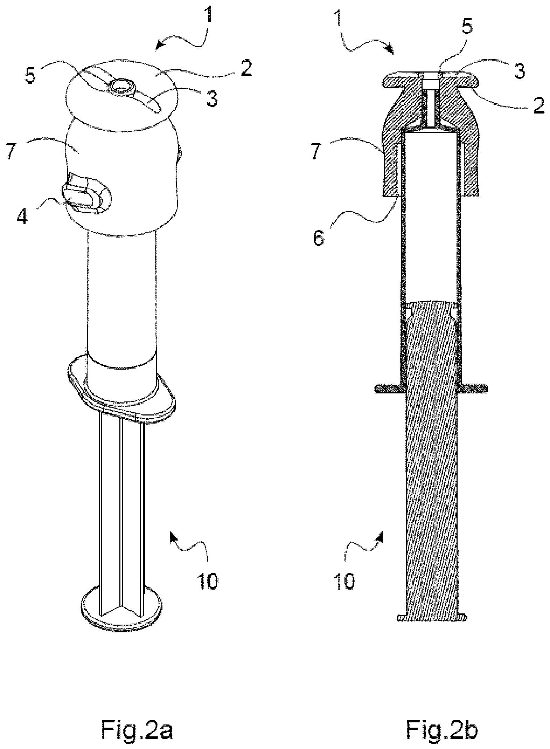

In figure 2a can be appreciated a conventional syringe (10) with a nozzle (1) according to the preferred embodiment connected to its mouth or outlet portion, which it completely covers. Preferably, the syringe (10) is filled with a mucus clearing fluid, preferably a saline solution, and the second end of the nozzle is then connected to the outlet portion of the syringe (10), devoid of a hypodermic needle. Thus, the nozzle (1) is pushed with the fingers by pressing on the two flanges (4) arranged in radially opposite positions on the side wall (7) of the nozzle (1). In this way, the nozzle (1) is tightly connected to the syringe (10). In use, a syringe (10) filled with saline solution has to be placed near the user's nose and with the nozzle (1) connected, establishing contact between the rim (2) and the outside of the user's nose and pressing the syringe plunger (10) to place the fluid inside the user's nostril.

Fig. 2b shows a section view of the syringe (10) with a nozzle (1) according to the preferred embodiment of the invention shown in Fig. 2a, in which the placement of the outlet portion of the syringe (10) into the nozzle (1) can be seen.

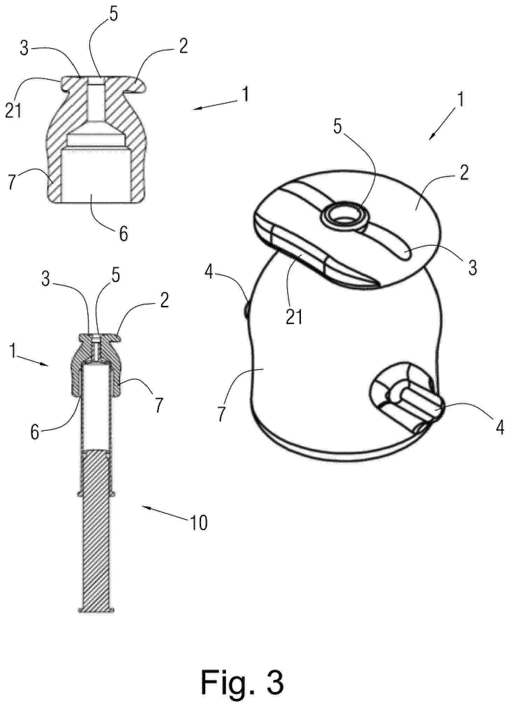

In another example of embodiment of the dosing nozzle (1), as shown in figure 3, the rim (2) of the nozzle (1) has a recess (21) in a portion of the outer edge of the rim (2). In the example shown, the rim (2) is circular, and the recess (21) is made as a cut or section along a chord of the circle that defines the rim (2). The recess (21) is adapted to facilitate the support of the nozzle (1), settling in the philtrum or subnasal groove; Furthermore, the recess (21) comprises a slightly undulating section adapted to better follow the contour of the user's subnasal groove.

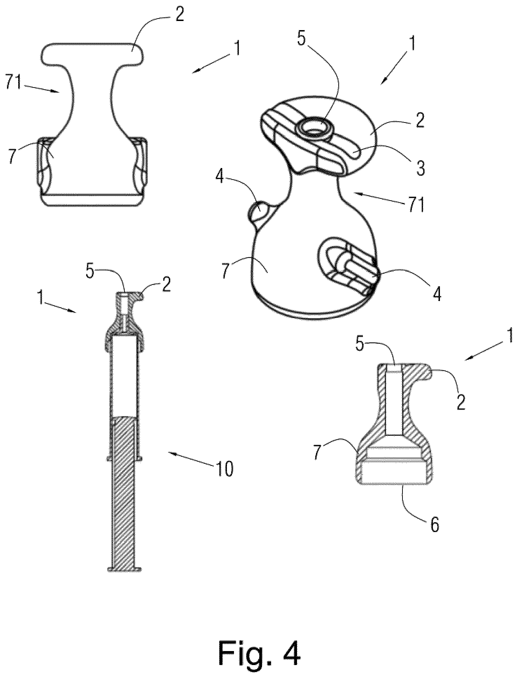

In another example of embodiment of the dosing nozzle (1), shown in Fig. 4, the side wall (7) also comprises a narrowing (71) between the rim (2) and the side wall portion (7) wherein the flanges (4) are arranged. This narrowing (71) allows the nozzle (1) to be adapted to the anatomy of the nostrils of babies, whose noses are smaller than those of adults.

## Claims

1. Dispensing nozzle (1) configured for applying of a pressurized fluid from a syringe (10) inside a nasal cavity, where the nozzle (1) has a substantially tubular configuration, and comprises:
a first end with at least a first opening (5),
a second end with at least a second opening (6), and
a side wall (7) extending between the first end and the second end, where the first opening (5) and the second opening (6) they are in fluid communication with each other;
the nozzle (1) being **characterized in that**:
the first end comprises an elastically deformable rim (2) arranged around the first opening (5), and configured to establish a tight seal between the first end and the outside of the nose,
the rim (2) comprises at least one slit (3) extending between the first opening (5) and an outer edge of the rim (2), the slit (3) being configured to relieve an excess pressure inside the nasal cavity,
the second end is elastically deformable and is configured to connect the second opening (6) tightly with an outlet portion of a syringe (10), and
the side wall (7) comprises at least one flange (4) configured to push or pull the nozzle (1) in order to connect or disconnect the second opening (6) to the outlet portion of the syringe.

2. Dispensing nozzle (1) according to the previous claim, wherein the rim (2) is separable from the rest of the nozzle (1) and interchangeable with rims (2) of different sizes and shapes.

3. Dispensing nozzle (1) according to claim 1, wherein the nozzle (1) is formed by an integral piece.

4. Dispensing nozzle (1) according to any of the preceding claims, wherein the nozzle (1) has substantially axial symmetry with respect to a longitudinal axis that passes through the first opening (5) and the second opening (6).

5. Dispensing nozzle (1) according to any of the preceding claims, wherein the nozzle (1) comprises two substantially elongated flanges (4) arranged perpendicularly or obliquely with respect to the longitudinal axis of the nozzle (1).

6. Dispensing nozzle (1) according to any of claims 3-5, wherein the nozzle (1) is made up of an elastically deformable material.

7. Dispensing nozzle (1) according to the previous claim, wherein the nozzle (1) is made up of rubber, food grade silicone or natural rubber.

8. Dispensing nozzle (1) according to any of the preceding claims, wherein the rim (2) comprises a recess (21) in a portion of its outer edge.

9. Dispensing nozzle (1) according to claim 1, wherein the side wall (7) comprises a narrowing (71).
